# EUROPÄISCHE PATENTSCHRIFT

(11) **EP 2 386 266 B1**
(45) Veröffentlichungstag und Bekanntmachung des Hinweises auf die Patenterteilung: **07.11.2012**
(21) Anmeldenummer: 11001175.6
(22) Anmeldetag: 14.02.2011
(51) Int. Cl.: A61F 2/00, A61F 2/18

(54) **Transportvorrichtung für Gehörknöchelchenprothesen**
Transport device for ossicle prosthetics
Dispositif de transport pour prothèses d'osselets d'oreille

(30) Priorität: 12.05.2010 DE 202010006737 U; 24.06.2010 DE 102010024894
(43) Veröffentlichungstag der Anmeldung: 16.11.2011
(73) Patentinhaber: Heinz Kurz GmbH Medizintechnik, 72144 Dusslingen (DE)
(72) Erfinder: Steinhardt, Uwe, 72145 Hirrlingen (DE); Gamer, Walter, 76297 Stutensee (DE)
(74) Vertreter: Kohler Schmid Möbus

(56) Entgegenhaltungen:
- DE-U1-202005 015 801
- US-A- 4 288 066
- US-A- 5 669 501

## Beschreibung

Die Erfindung betrifft eine Transportvorrichtung, die für eine Aufnahme und gegen Herausfallen gesicherte Halterung einer Gehörknöchelchenprothese zur Implantation ins Mittelohr während des Transports zum Operateur eingerichtet ist, wobei die Gehörknöchelchenprothese mindestens Teile eines Gliedes der menschlichen Gehörknöchelchenkette ersetzen oder überbrücken kann.

Eine derartige Transportvorrichtung für Gehörknöchelchenprothesen ist bekannt aus der US-A 4,288,066.

Gehörknöchelchenprothesen werden verwendet, um bei ganz oder teilweise fehlenden oder geschädigten Gehörknöchelchen des menschlichen Mittelohrs den Schall bzw. das Schallsignal vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Drei besonders häufig verwendete Arten von Gehörknöchelchenprothesen sind die Steigbügel-Prothesen, die Partial-Prothesen und die Total-Prothesen. Steigbügel-Prothesen (= Stapes-Prothesen) werden am Amboss fixiert und ragen über einen Stempel (= Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Da Gehörknöchelchenprothesen naturgemäß mit ihren Außenabmessungen im Bereich von wenigen Millimetern liegen und in ihrem funktionellen Feinaufbau sehr fragil sind, müssen gattungsgemäße Transportvorrichtungen, mit denen derartig zerbrechliche Prothesen vom Hersteller zum Operateur transportiert werden, nicht nur den hygienischen Anforderungen an eine sterile Verpackung genügen, sondern auch sicherstellen, dass die jeweilige Prothese unbeschädigt ankommt.

Denkbar für diesen Zweck ist der Einsatz von Frästeil-Halterungen oder Spritzgussteilen aus Vollmaterial, die Aushöhlungen von geeigneter Form und Größe enthalten, um die Gehörknöchelchenprothese für den Transport aufzunehmen, jedoch bei der Herstellung relativ hohe Kosten verursachen.

Aus der US-A 5,669,501 ist eine Transportvorrichtung für eine Gehörknöchelchenprothese bekannt, die eine flache Grundplatte in Form einer Bodenplatte eines Einsatzes aufweist, in die eine Einhängevorrichtung für die Aufnahme der Gehörknöchelchenprothese integriert ist.

Die eingangs zitierte US-A 4,288,066 beschreibt einen Behälter für eine Mittelohrprothese, der eine sowohl zum Transport der Prothese zum Operateur als auch zu deren mechanischer Bearbeitung durch den Operateur geeignet sein soll. Diese bekannte Transportvorrichtung besteht aus einem - relativ dicken - Spritzguss-Behälter aus Kunststoff mit geeigneter Ausnehmung zum Einlegen der Prothese sowie einem mittels einer Lasche verklappbar an dem Behälter anhängenden Deckel zum Verschließen des Behälters. Die Ausnehmung ist so gestaltet, dass die Mittelohrprothese vor der Implantation, aber noch unmittelbar vor ihrer Entnahme aus dem Behälter auf ein gewünschtes Längenmaß gekürzt werden kann.

Nachteilig bei diesem bekannten Behälter sind einmal seine - im Verhältnis zur Prothesengröße - relativ sperrigen Abmaße, sein komplizierter innerer Aufbau und die daraus resultierenden Kosten für das entsprechende Fertigungswerkzeug sowie die Festlegung auf jeweils genau eine bestimmte Prothesenform und -größe durch die vom Spritzgusswerkzeug für den Behälter vorgegebene Geometrie.

Aufgabe der vorliegenden Erfindung ist es demgegenüber, mit möglichst einfachen technischen Mitteln unaufwändig und kostengünstig eine Transportvorrichtung für Gehörknöchelchenprothesen der eingangs beschriebenen Art bereit zu stellen, die sehr leicht und in ihren Abmaßen möglichst kompakt ist, einfach herzustellen sein soll und dabei eine hohe Flexibilität in Bezug auf Größen und Geometrien der zu transportierenden Mittelohrprothese erlaubt, wobei einerseits eine sichere Halterung der Prothese während des Transports gewährleistet ist und andererseits die Prothese vom Operateur vor der Entnahme noch innerhalb der Transportvorrichtung bequem bearbeitbar ist. Erfindungsgemäß wird diese Aufgabe auf ebenso überraschend einfache wie wirkungsvolle Weise dadurch gelöst, dass die Transportvorrichtung eine flache Grundplatte aus dünnem Metallblech mit einer Dicke von etwa einem halben Millimeter umfasst, in die mindestens eine, vorzugsweise zwei mit der Grundplatte über feine Stege verwindbar verbundene Einhängevorrichtungen für die Aufnahme und Halterung der Gehörknöchelchenprothese integriert sind, wobei die Gehörknöchelchenprothese an ihrem einen Ende ein erstes Befestigungselement zur mechanischen Verbindung mit dem Trommelfell oder einem Glied der Gehörknöchelchenkette und an ihrem anderen Ende ein zweites Befestigungselement zur mechanischen Verbindung mit einem weiteren Glied oder Teilen eines Gliedes der Gehörknöchelchenkette oder direkt mit dem Innenohr sowie ein die beiden Befestigungselemente Schall leitend miteinander verbindendes Verbindungselement aufweist, und dass die Einhängevorrichtung bei der Fertigung der Transportvorrichtung mit der Oberfläche der Grundplatte fluchtet und zum Einhängen der Gehörknöchelchenprothese gegenüber diesem flach liegenden Fertigungs-Zustand mittels einer plastischen Verformung der Stege um etwa 90° gegenüber der Grundplatte verdreht werden kann und dann über die Oberfläche der Grundplatte herausragt.

Die erfindungsgemäße Transportvorrichtung lässt sich in Außenabmessungen herstellen, die kaum größer sind als die der aufzunehmenden Gehörknöchelchenprothese, wobei das Gewicht der Vorrichtung - abhängig von der Wahl des Ausgangsmaterials - ebenfalls in die Größenordnung des Prothesengewichts kommt. Die verschwenkbaren Einhängevorrichtungen können ohne größeren Fertigungsaufwand leicht so gestaltet werden, dass auch Mittelohrprothesen unterschiedlicher Größen und Geometrien sicher aufgenommen und gehaltert werden können, wobei durch die Verdrehung um ca. 90° gegenüber der Grundplatte nach dem Transport und vor der Entnahme der Prothese eine ergonomisch günstige Bearbeitungsmöglichkeit geboten wird.

Die erfindungsgemäße Transportvorrichtung wird besonders bevorzugt durch Bearbeitung der Grundplatte mittels Laserbehandlung und/oder Anodisierung mit hoher Genauigkeit der zu erzeugenden feinen Strukturen hergestellt.

Ganz besonders günstig herstellbar sind Ausführungsformen der Erfindung, bei denen die Grundplatte und die Einhängevorrichtung gemeinsam aus einem zusammen hängenden Nutzen gefertigt sind.

Eine Klasse von weiteren vorteilhaften Ausführungsformen der Erfindung zeichnet sich dadurch aus, dass die Transportvorrichtung eine während des Transports die Grundplatte allseitig umhüllende Umverpackung, insbesondere in Form eines Behälters wie einer Schachtel, einer Dose oder eines Kästchens umfasst.

Vorteilhaft im Hinblick auf einen beschädigungsfreien Transport der Gehörknöchelchenprothesen sind Weiterbildungen dieser Ausführungsformen, bei denen die Grundplatte eine Einrichtung zur Transportsicherung aufweist, welche seitlich von der Grundplatte abstehende grätenförmige Stege umfasst, die sich gegen Innenwände der Umverpackung verklemmen oder verspannen lassen.

Die Befestigung von Gehörknöchelchenprothesen, für welche die gattungsgemäße Transportvorrichtung vorgesehen ist, kann an einem Glied der Gehörknöchelchenkette während der Implantation dadurch bewirkt oder zumindest unterstützt wird, dass die gesamte Prothese oder wenigstens das entsprechende Befestigungselement aus einem Material mit Formgedächtnis (memory effect) - in der Regel die Nickel-Titan-Legierung Nitinol - hergestellt wurde, welches präoperativ einer Verformung unter definierten Temperaturbedingungen unterzogen wurde. Um nun ein beispielsweise als Schlinge ausgelegtes Befestigungselement dauerhaft zu befestigen, wird die Schlinge zunächst relativ grob um den winzigen Teilbereich des entsprechenden Gehörknöchelchens herum gelegt, genau positioniert und anschließend thermisch - meist mit einem Laser oder einer elektrischen Einrichtung - derart behandelt, dass durch gezielte Verformung des Memory-Materials ein endgültiger und - bei Körpertemperatur dann auch permanenter - Verschluss der Schlinge um das Knöchelchen herum erfolgt. Analog wird auch bei anders geformten Befestigungselementen vorgegangen.

Problematisch ist dabei jedoch, dass beim Einbringen von zu hoher Lichtleistung bzw. elektrischer Energie während der Implantation der Mittelohrprothese ungewollt eine zu große lokale Aufheizung und damit eine zu hohe Temperatur im Bereich des Befestigungselements erzeugt wird, die sich auf das Gehörknöchelchen überträgt und schnell zu einer Schädigung oder gar Nekrotisierung des empfindlichen Körpergewebes am entsprechenden Abschnitt des Gehörknöchelchens führen kann. Die ganze Operation wäre dann im Endergebnis möglicherweise sogar kontraproduktiv.

Natürlich machen die Hersteller von gattungsgemäßen Mittelohrprothesen in der Regel Angaben über optimale Verfahrensparameter bei der "Bearbeitung" ihrer Prothesen mit memory effect. Aber allein schon wegen der stark differierenden Geräte der Anwender zur thermischen Behandlung vor Ort können diese Angaben lediglich sehr breite und damit oftmals nicht sonderlich aussagekräftige Bereiche umfassen. Außerdem hilft es einem Operateur in der Praxis herzlich wenig, wenn ihm gesagt wird, welche lokale Maximaltemperatur er bei der thermischen Behandlung nicht überschreiten darf. Er weiß ohnehin, dass Eiweiß ab etwa 60°C koaguliert und Körpergewebe dauerhaft geschädigt werden kann. Vielmehr muss er eine geeignete Einstellung der Leistungsabgabe seines individuell vorhandenen Gerätes finden und außerdem auch die für seine Zwecke optimale Stelle der Energiezufuhr auf die Gehörknöchelchenprothese herausfinden, bevor er die Operation im Mittelohr durchführt.

Um dem Operateur eine unkomplizierte Möglichkeit zu eröffnen, intraoperativ kurz vor der eigentlichen Implantation der Mittelohrprothese eine für seine vorhandenen thermischen Behandlungsgeräte optimale Parametereinstellung zu ermitteln, ohne dass die Mittelohrprothese selbst auch nur berührt werden muss oder ungewünscht irgendwie verändert werden könnte, ist es vorteilhaft, wenn eine Vorrichtung zur Ermittlung einer optimierten Leistungseinstellung und/oder Energieapplikations-Stelle für die Bearbeitung der intraoperativ thermisch zu behandelnden Teile der Gehörknöchelchenprothese vorgesehen ist, die eine der Prothese nachgebildete Prothesenattrappe enthält, welche zumindest in denjenigen Bereichen, in denen die zugehörige Prothese intraoperativ thermisch behandelt werden soll, mit der Gehörknöchelchenprothese hinsichtlich Material, geometrischer Gestaltung und Herstellungsverfahren identisch aufgebaut ist.

Auf diese Weise kann der Operateur zunächst die optimalen Parameter der für die Gehörknöchelchenprothese hinterher in situ vorgesehenen thermischen Behandlung im Mittelohr bereits vorher an der Prothesenattrappe mit seinen vorhandenen Geräten ermitteln und geplante Handlungsabläufe statt im Mittelohr problemlos außerhalb austesten, ohne dabei die originale Prothese auch nur berühren zu müssen und dabei eventuell ungewollt zu verformen oder gar zu beschädigen. Die so ermittelten Parameter entsprechen sehr genau den für die Original-Prothese gültigen Parametern, weil die Prothesenattrappe zumindest an den Stellen der geplanten thermischen Behandlung mit der Original-Prothese identisch übereinstimmt.

Ganz besonders bevorzugt ist vor diesem Hintergrund eine Klasse von Ausführungsformen der erfindungsgemäßen Transportvorrichtung, die sich dadurch auszeichnen, dass die Transportvorrichtung eine Halterungsvorrichtung aufweist, welche auch die oben beschriebene Prothesenattrappe verschwenkbar aufnehmen kann.

Besonders bevorzugte und für den Benutzer komfortable Weiterbildungen dieser Ausführungsformen zeichnen sich dadurch aus, dass die Halterungsvorrichtung für die Prothesenattrappe eine mit der Grundplatte über feine Stege verwindbar verbundene weitere Einhängevorrichtung zum Einhängen eines Endes der Prothesenattrappe enthält. Damit kann nicht nur eine stabile und gut zugängliche Positionierung der Prothesenattrappe während der Ermittlung der optimalen Behandlungsparameter, sondern auch ein schonender und beschädigungsfreier Transport zum "Einsatzort" sichergestellt werden.

Eine weitere erhebliche Verbesserung der Handhabung wird bei Varianten der obigen Ausführungsformen erreicht, bei welchen die Prothesenattrappe eine Einrastvorrichtung enthält, mit welcher die Prothesenattrappe zumindest in einer Bearbeitungsposition in der Grundplatte fixierbar ist. Die starre und unverrückbare Lage der Prothesenattrappe während der Ermittlung von optimalen Parametern erleichtert nicht nur die Arbeit, sondern erhöht auch die Qualität der aufgefundenen Parameter.

Zum Schutz der besonders empfindlichen Abschnitte von Mittelohrprothese und zugehöriger Prothesenattrappe ist bei bevorzugten Weiterbildungen vorgesehen, dass in die Grundplatte einsteckbare oder in der Grundplatte integrierte Platzhalter-Pins vorhanden sind, die so geformt sind, dass sie die thermisch vorbehandelten, aus Material mit Formgedächtnis bestehenden Teile der Gehörknöchelchenprothese sowie gegebenenfalls die entsprechenden Abschnitte der zugehörigen Prothesenattrappe während des Transports zum Operateur in der gewünschten Geometrie derart fixieren, dass auch bei einer eventuellen Erwärmung während des Transports ungewünschte Formveränderungen verhindert werden.

Besonders bevorzugt sind auch Ausführungsformen der erfindungsgemäßen Transportvorrichtung, bei denen die Grundplatte optisch kodiert und/oder beschriftet ist, wobei die Kodierung oder Beschriftung technische Informationen bezüglich der Gehörknöchelchenprothese und/oder einer zugehörigen Prothesenattrappe und/oder Herstellerangaben enthält, so dass diese wichtigen Daten nicht erst in einem Handbuch oder Begleitzettel zur Transportvorrichtung nachgeschlagen werden müssen, sondern unmittelbar bei der Handhabung der Transportvorrichtung zur Verfügung stehen.

Von besonderem praktischen Vorteil schließlich sind Weiterbildungen, dieser Ausführungsformen, bei denen die technischen Informationen der Kodierung und/oder Beschriftung einen empfohlenen Anfangswert für die elektrische Leistung oder Lichtleistung enthalten, welcher zu Beginn der Ermittlung von optimierten Bearbeitungsparametern der intraoperativ thermisch zu behandelnden Teile der Gehörknöchelchenprothese mit Hilfe einer Testbehandlung der Prothesenattrappe angewendet werden soll. Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Im Einzelnen zeigen:
- Fig. 1: eine Ausführungsform der erfindungsgemäßen Transportvorrichtung mit einer neben einer zugehörigen Gehörknöchelchenprothese auf einer Halterungsvorrichtung im Bereich einer Einrastvorrichtung verschwenkbar aufgenommenen Prothesenattrappe in einer schematischen räumlichen Darstellung von schräg oben;
- Fig. 2a: eine Detailansicht der Prothesenattrappe im Bereich der Einrastvorrichtung bei der Ausführungsform von Fig. 1;
- Fig. 2b: eine Detailansicht mit der gesamten Prothesenattrappe in einer schematischen räumlichen Darstellung von schräg oben um etwa 180° gegenüber Fig. 1 verdreht;
- Fig. 3a-3e: die Ausführungsform von Fig. 1 in schematischer Draufsicht
(a) von oben,
(b) von unten,
(c) von einer Stirnseite I,
(d) von der Gegen-Stirnseite II und
(e) von einer Längsseite III;
- Fig. 4a: die Ausführungsform von Fig. 1 in schematischer räumlicher Darstellung von schräg oben mit entnommener Gehörknöchelchenprothese und aufgerichteter und mit der Halterungsvorrichtung verrasteter Prothesenattrappe;
- Fig. 4b: wie Fig. 4a, aber um etwa 180° verdreht aus der Perspektive der gegenüber liegenden Längsseite;
- Fig. 5a: die Grundplatte der Ausführungsform von Fig. 1 ohne Gehörknöchelchenprothese und ohne Prothesenattrappe sowie mit der verschwenkbaren Halterung für die Prothese und der Einhängevorrichtung für die Attrappe jeweils im unverschwenkten, flach liegenden Fertigungs-Zustand;
- Fig. 5b: die Transportvorrichtung von Fig. 5a in einer schematischen Ansicht von unten;
- Fig. 5c: die Transportvorrichtung von Fig. 5a mit der Einhängevorrichtung für die Gehörknöchelchenprothese im verschwenkten Zustand zur Aufnahme der zu transportierenden Prothese; und
- Fig. 5d: die Transportvorrichtung von Fig. 5c in einer schematischen Ansicht von unten.

Die in den Figuren der Zeichnung schematisch dargestellte Ausführungsform der erfindungsgemäßen **Transportvorrichtung 1** mit einer **Gehörknöchelchenprothese 2** zur Implantation ins Mittelohr, die an ihrem einen Ende ein **erstes Befestigungselement 2a** zur mechanischen Verbindung mit dem Trommelfell oder einem Glied der Gehörknöchelchenkette und an ihrem anderen Ende ein **zweites Befestigungselement 2b** zur mechanischen Verbindung mit einem weiteren Glied der Gehörknöchelchenkette oder direkt mit dem Innenohr sowie ein die beiden Befestigungselemente 2a, 2b Schall leitend miteinander verbindendes **Verbindungselement 2c** aufweist, und wobei zumindest Teile der Gehörknöchelchenprothese 2 aus einem Material mit Formgedächtnis (= memory effect) hergestellt sind, welche bei der Implantation der Gehörknöchelchenprothese 2 im Mittelohr einer Form verändernden thermischen Behandlung unterzogen werden, zeichnet sich erfindungsgemäß dadurch aus, dass die Transportvorrichtung 1 eine flache **Grundplatte 5 -** in der Regel aus dünnem Metallblech von geringer Dicke - umfasst, in die mindestens eine mit der Grundplatte 5 über feine **Stege 6b'** verwindbar verbundene **Einhängevorrichtung 6b** für die Aufnahme und Halterung der Gehörknöchelchenprothese 2 integriert ist, und dass die Einhängevorrichtung 6b bei der Fertigung der Transportvorrichtung 1 mit der Oberfläche der Grundplatte 5 fluchtet und zum Einhängen der Gehörknöchelchenprothese 2 gegenüber diesem flach liegenden Fertigungszustand mittels einer plastischen Verformung der Stege 6b' um etwa 90° gegenüber der Grundplatte 5 verdreht werden kann und dann über die Oberfläche der Grundplatte 5 herausragt.

Die Transportvorrichtung 1 wird günstigerweise durch Bearbeitung der Grundplatte 5 mittels Laserbehandlung und/oder Anodisierung gemeinsam mit der Einhängevorrichtung 6b aus einem zusammen hängenden Nutzen hergestellt.

Des Weiteren trägt die erfindungsgemäße Transportvorrichtung 1 auch eine der Gehörknöchelchenprothese 2 nachgebildete **Prothesenattrappe 3,** die Teil einer Testeinrichtung zur Ermittlung einer optimierten Leistungseinstellung und/oder Energieapplikationsstelle für die Bearbeitung von intraoperativ thermisch zu behandelnden Teilen der Gehörknöchelchenprothese 2 ist. Diese Prothesenattrappe 3 ist zumindest in denjenigen Bereichen, in denen die zugehörige Prothese 2 intraoperativ thermisch behandelt werden soll, mit der Gehörknöchelchenprothese 2 hinsichtlich Material, geometrischer Gestaltung und Herstellungsverfahren identisch aufgebaut.

Die Gehörknöchelchenprothese 2 sowie ihre zugehörige Prothesenattrappe 3 sind bei der in Zeichnung gezeigten Ausführungsform der erfindungsgemäßen Transportvorrichtung 1 zumindest im Bereich des ersten Befestigungselements 2a aus einem Material mit Formgedächtnis hergestellt. Die Prothese kann auch eine Einrichtung zur Variation ihrer Länge aufweisen, wobei dann die Prothese sowie ihre zugehörige Prothesenattrappe zumindest im Bereich der Einrichtung zur Längenvariation aus einem Material mit Formgedächtnis hergestellt sind.

Als bevorzugtes Material mit Formgedächtnis (= memory effect) kommt bei vielen Gehörknöchelchenprothesen sowie gegebenenfalls den zugehörigen Prothesenattrappen eine Nickel-Titan-Legierung, insbesondere Nitinol zum Einsatz.

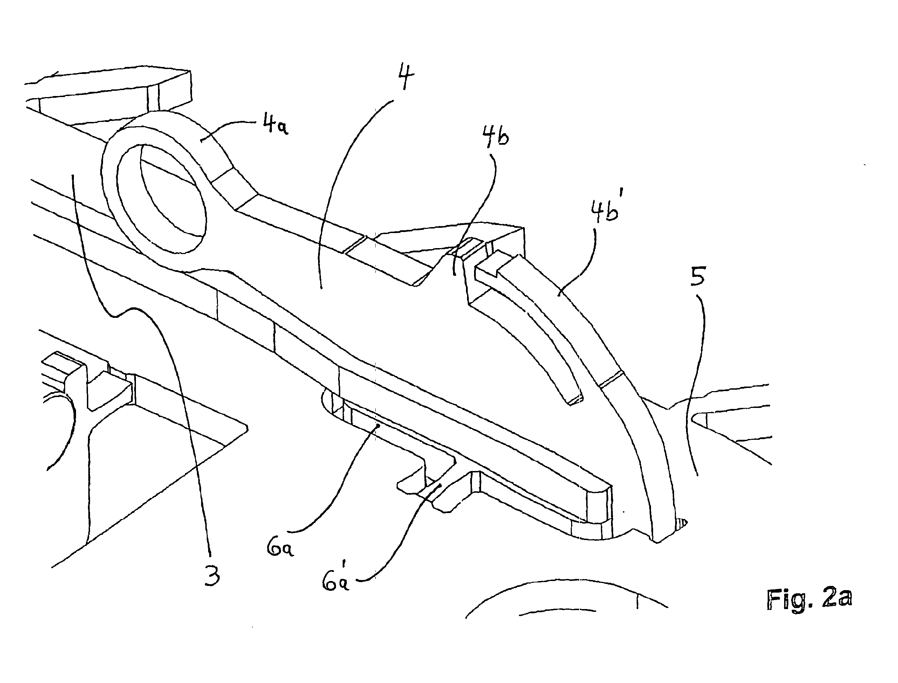

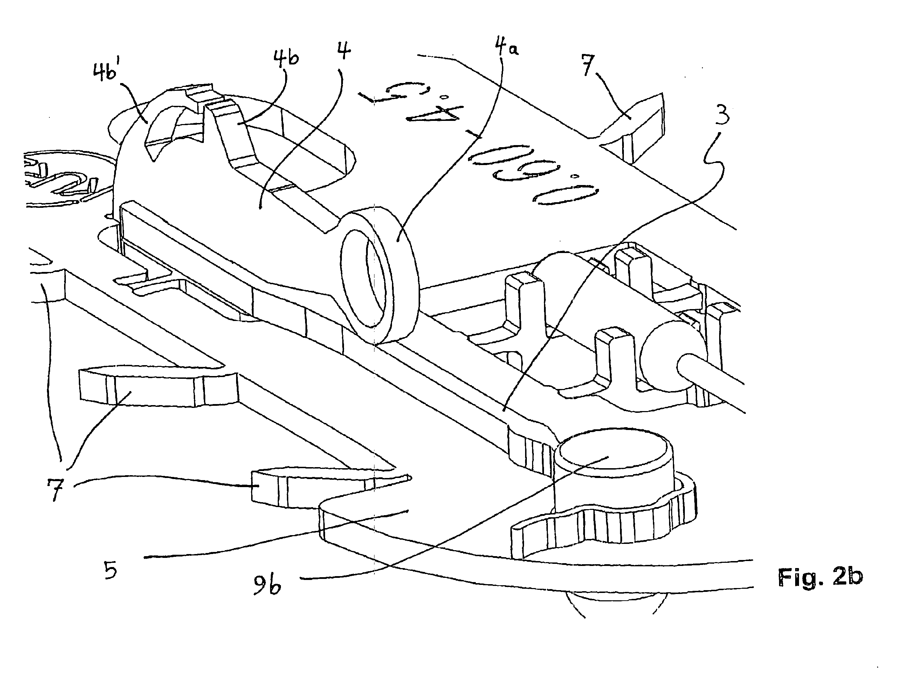

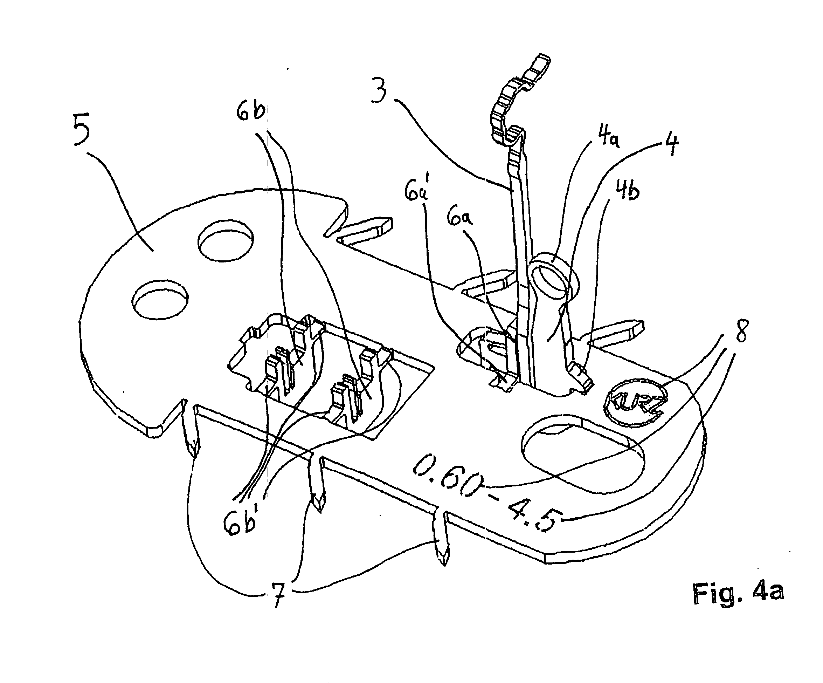

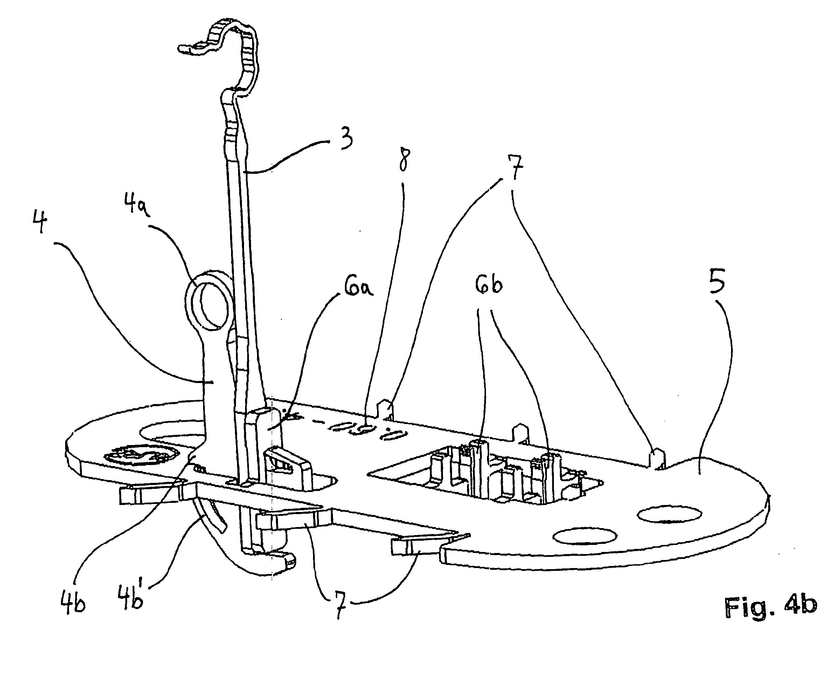

Bei allen in der Zeichnung gezeigten Darstellungen der Prothesenattrappe 3 ist eine **Einrastvorrichtung 4** vorgesehen, mit welcher die Prothesenattrappe 3 in einer - wie in den Figuren 4a und 4b zu erkennen vorzugsweise aufrechten - Bearbeitungsposition fixierbar ist. Mögliche Details der Einrastvorrichtung 4 sind in den Figuren 2a und 2b vergrößert dargestellt, etwa ein laschenförmiges **Bedienelement 4a** oder **Verriegelungselemente 4b, 4b',** die im fixierten Zustand eine Gegenhalterung umgreifen, wie in Fig. 4b gut zu erkennen ist.

Sämtliche in der Zeichnung dargestellten Ausführungsformen der erfindungsgemäßen Transportvorrichtung 1 stimmen auch darin überein, dass eine Halterungsvorrichtung vorgesehen ist, welche die Prothesenattrappe 3 verschwenkbar aufnehmen kann.

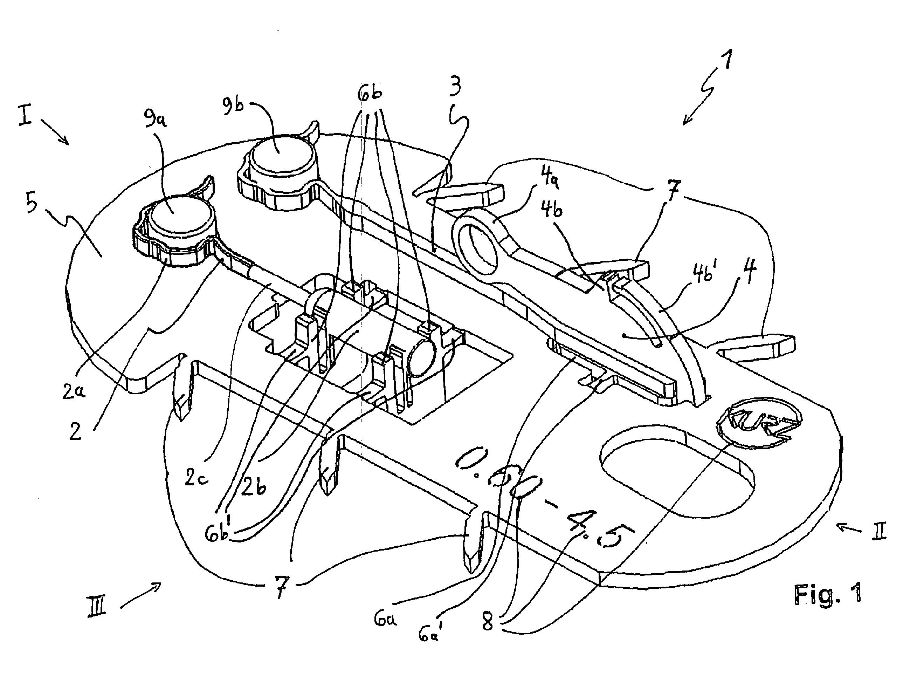

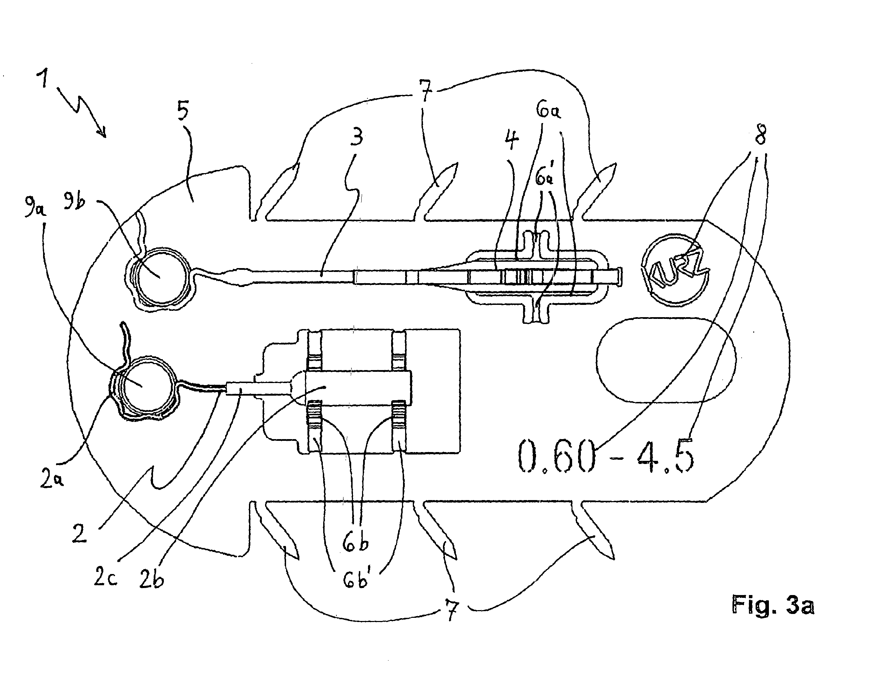

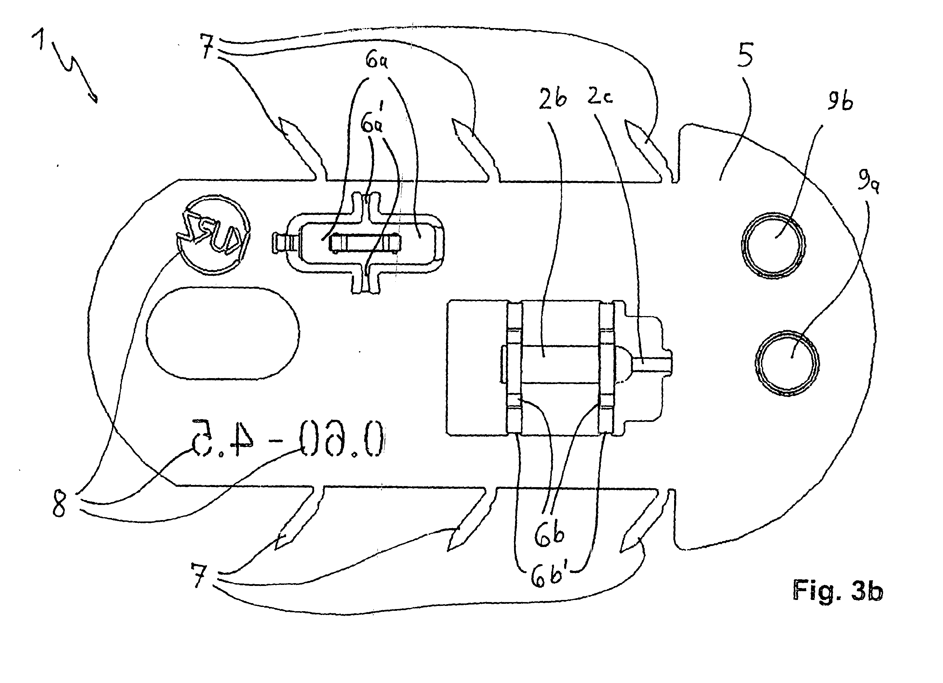

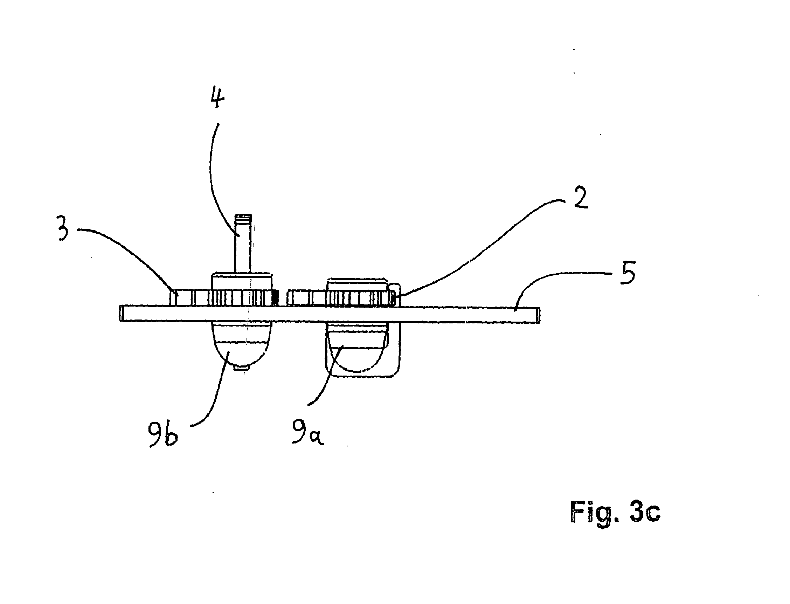

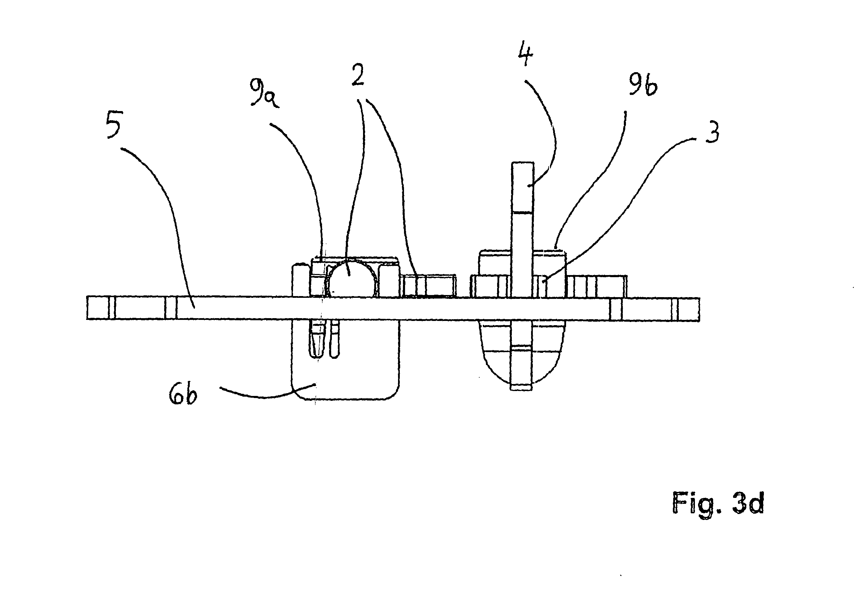

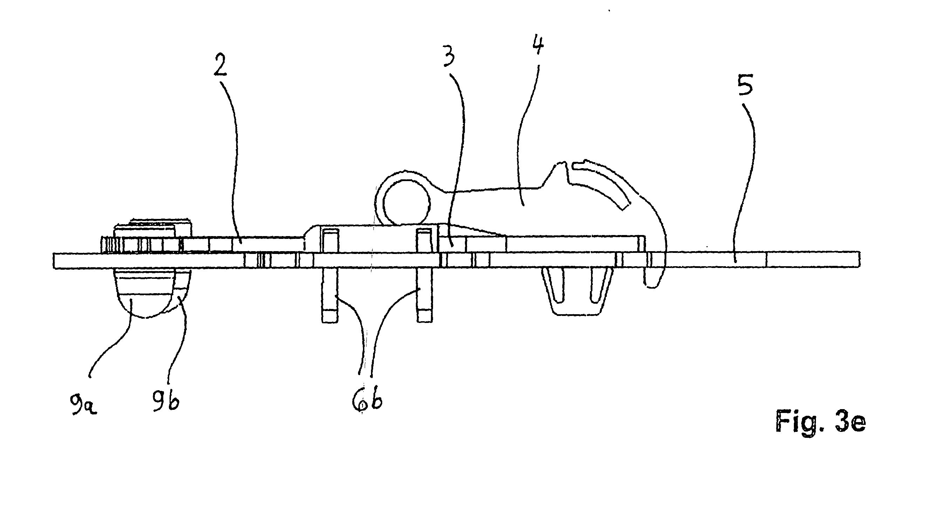

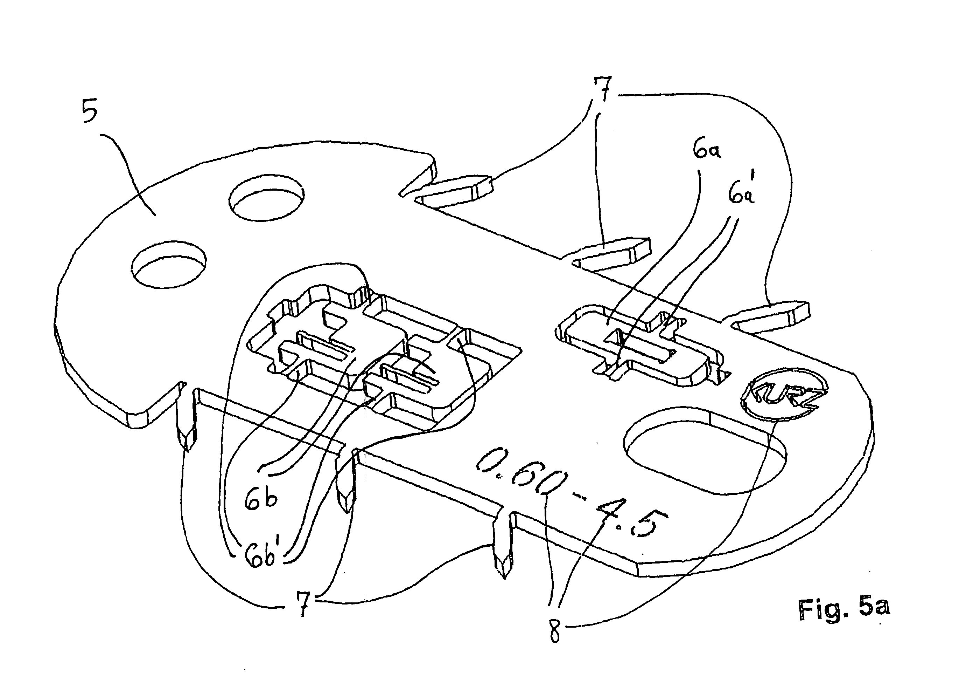

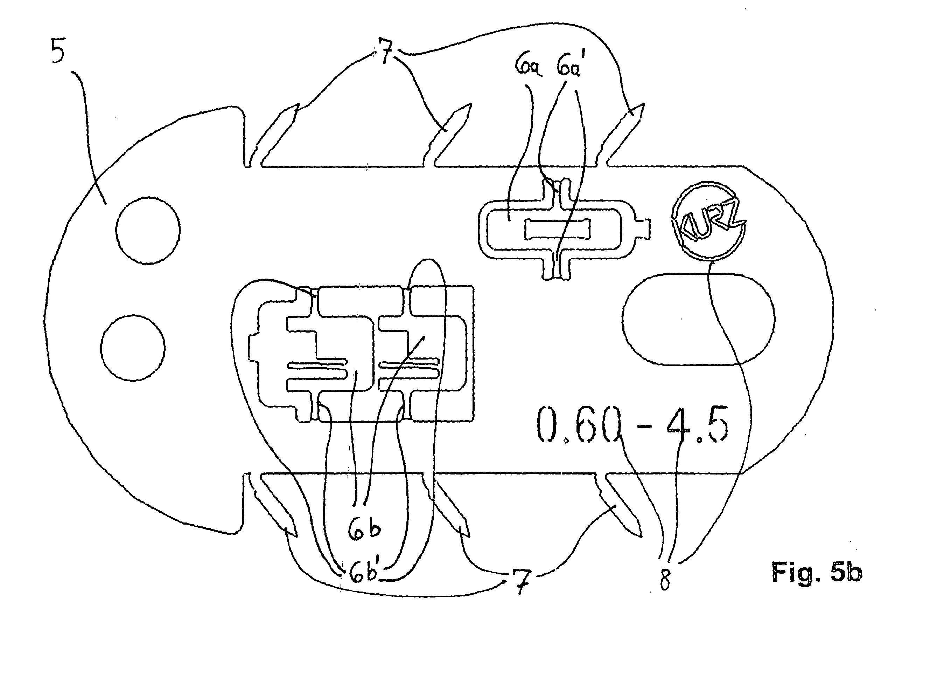

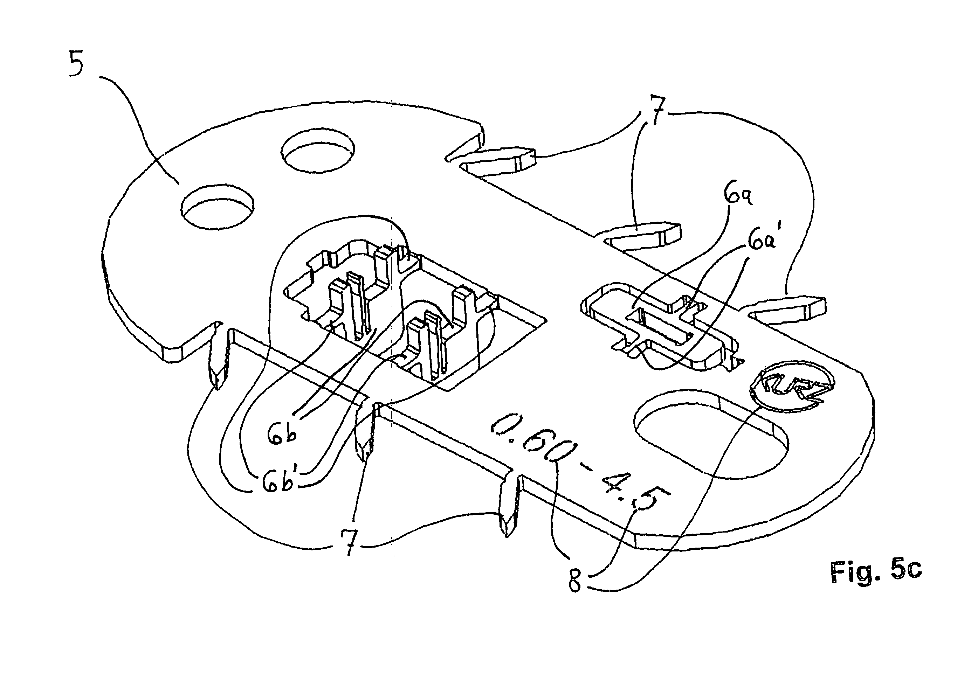

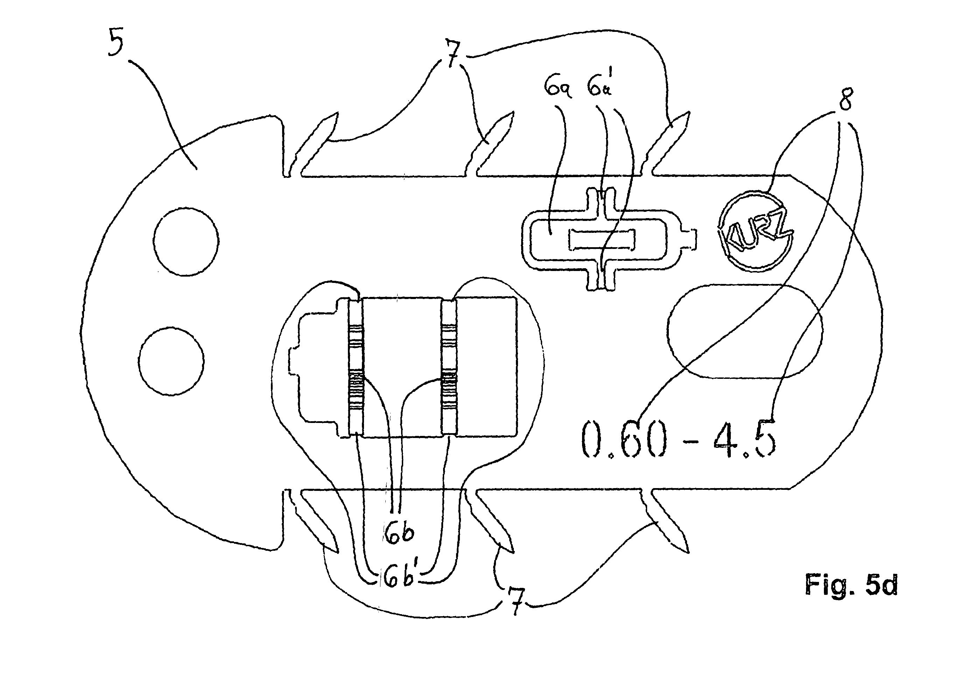

Die Figuren 1 sowie 3a bis 3e zeigen die Grundplatte 5 mit montierter Gehörknöchelchenprothese 2 und Prothesenattrappe 3 im flach liegenden Transport-Zustand, die Figuren 4a und 4b mit aufgestellter und verrasteter Prothesenattrappe 3 bei bereits entnommener Prothese 2 und die Figuren 5a bis 5d ohne Prothesenattrappe 3 und Prothese 2, wobei die Figuren 5a und 5b speziell den Zustand der Halterungsvorrichtung 5 unmittelbar nach deren Fertigung darstellen, während die Figuren 5c und 5d den Zustand kurz vor der Montage der Gehörknöchelchenprothese 2 auf der Halterungsvorrichtung 5 illustrieren. Nicht dargestellt sind mögliche Ausführungsformen der erfindungsgemäßen Transportvorrichtung, bei welchen die Halterungsvorrichtung derart gestaltet ist, dass sie nur die Prothesenattrappe, nicht aber die zugehörige Gehörknöchelchenprothese (oder vice versa) aufnehmen kann.

Die Figuren der Zeichnung zeigen eine besonders bevorzugte Ausführungsform der Erfindung, bei der die Halterungsvorrichtung für die Prothesenattrappe 3 eine mit der Grundplatte 5 über feine **Stege 6a'** verwindbar verbundene **Einhängevorrichtung 6a** zum Einhängen eines Endes der Prothesenattrappe 3 umfasst. Wie oben erwähnt, illustrieren Figuren 5c und 5d insbesondere den Zustand der Transportvorrichtung 1 kurz vor der Montage der Gehörknöchelchen-prothese 2, wobei die Einhängevorrichtungen 6b für die Aufnahme der Prothese 2 gegenüber dem flach liegenden Zustand in den Figuren 5a und 5b mit Hilfe der Stege 6b' um etwa 90° gegenüber der Grundplatte 5 verdreht sind. Die Prothesenattrappe 3 hingegen wird für den Transport der Testeinrichtung zum Operateur im unverdrehten Zustand der Einhängevorrichtung 6a eingehängt und erst zur Ermittlung der optimalen Bearbeitungsparameter der Gehörknöchelchenprothese 2 gegenüber der Platte um etwa 90° verschwenkt und verrastet, wie in den Figuren 4a und 4b gut zu erkennen ist.

Die Transportvorrichtung 1 wird in der Regel eine während des Transports die Grundplatte 5 allseitig umhüllende Umverpackung, insbesondere in Form eines Behälters wie einer Schachtel, einer Dose oder eines Kästchens umfassen, die in der Zeichnung nicht eigens dargestellt ist.

Des Weiteren weist die Transportvorrichtung 1 eine Einrichtung zur Transportsicherung auf, welche seitlich von der Grundplatte 5 in der Grundplattenebene abstehende **grätenförmige Stege 7** umfasst, die bei - nicht dargestellten - Ausführungsformen der Erfindung aber auch schräg aus der Grundplattenebene herausragen können. Diese Stege 7 dienen für die Transportvorrichtung 1 mit der aufmontierten, gegen mechanische Beschädigungen relativ empfindlichen Gehörknöchelchen-prothese 2 und der gegebenenfalls ebenfalls aufmontierten Prothesenattrappe 3 als Verrutschsicherung während des Transports zum Operateur, wobei sich die Stege 7 gegen Innenwände der oben erwähnten Umverpackung verklemmen oder verspannen lassen.

Die Grundplatte 5 ist bei der gezeigten Ausführungsform der Erfindung optisch kodiert bzw. beschriftet. Die **Kodierung oder Beschriftung 8** enthält technische Informationen bezüglich der Gehörknöchelchen-prothese 2 und/oder der zugehörigen Prothesenattrappe 3 sowie Herstellerangaben. Die Kodierung kann auch eine einfache Farbkodierung umfassen. Alternativ oder ergänzend dazu können - bei in der Zeichnung nicht dargestellten Ausführungsformen - auch die Gehörknöchelchenprothese und/oder die zugehörige Prothesenattrappe optisch kodiert und/oder beschriftet sein, wobei die Kodierung oder Beschriftung wiederum technische Informationen über die Gehörknöchelchenprothese und/oder der Prothesenattrappe enthält.

Die technischen Informationen der Kodierung bzw. Beschriftung 8 enthalten unter anderem neben der einer Größenklassifizierung der verwendeten Gehörknöchelchenprothese 2 einen empfohlenen Anfangswert für die elektrische Leistung oder Lichtleistung, welcher zu Beginn der Ermittlung von optimierten Bearbeitungsparametern der intraoperativ thermisch zu behandelnden Teile der Gehörknöchelchenprothese 2 mit Hilfe einer Testbehandlung der Prothesenattrappe 3 angewendet werden soll.

Die Kodierung und/oder Beschriftung 8 wird am einfachsten mittels Laserbehandlung und/oder Anodisierung erzeugt. Ebenso wird die Grundplatte 5 selbst bevorzugt durch Laserschneiden der Platte aus einem größeren Blech hergestellt und weiterbearbeitet. Insbesondere die winzigen Strukturen der oben beschriebenen Elemente der Grundplatte 5, wie etwa der Einhängevorrichtungen 6a und 6b, der zugehörigen verwindbaren Stege 6a' und 6b' sowie der grätenförmige Stege 7 für die Transportsicherung lassen sich mit vertretbarem Aufwand kaum auf andere Weise herstellen.

Zum definierten Offenhalten des ersten Befestigungselements 2a der Gehörknöchelchenprothese 2 sowie des entsprechenden Abschnitts der zugehörigen Prothesenattrappe 3 während des Transports zum Operateur sind bei der gezeigten Ausführungsform der erfindungs-gemäßen Transportvorrichtung 1 **Platzhalter-Pins 9a, 9b** in der Grundplatte 5 vorgesehen, welche die thermisch vorbehandelten, aus Material mit Formgedächtnis bestehenden Teile der Gehörknöchelchenprothese 2 und gegebenenfalls der Prothesenattrappe 3 in der gewünschten Geometrie fixieren, sodass auch bei einer eventuellen Erwärmung während des Transports ungewünschten Formveränderungen mit Sicherheit verhindert werden können.

Die in den Figuren der Zeichnung dargestellte Ausführungsform der erfindungsgemäßen Transportvorrichtung 1 trägt eine Mittelohrprothese 2, bei welcher das erste Befestigungselement 2a die Form einer Klammer aufweist, die beispielsweise auf den Ambossfortsatz oder auch auf ein anderes Glied der Gehörknöchelchenkette aufgeklipst werden kann. Das zweite Befestigungselement 2b an dem der Klammer entgegen gesetzten Ende ist in diesem Ausführungsbeispiel als Kolben (= Piston) zur direkten Ankopplung der Gehörknöchelchenprothese 2 an das Innenohr ausgebildet.

Bei in der Zeichnung nicht dargestellten Ausführungsformen der Erfindung kann die Transportvorrichtung aber auch so ausgebildet sein, dass sie eine Gehörknöchelchenprothese aufnehmen kann, deren Befestigungselemente geometrisch anders gestaltet sind, etwa als Hülse, Schlinge oder Haken. Das erste Befestigungselement kann eine Kopfplatte zur Anlage am Trommelfell sein. Auch kann beispielsweise das zweite Befestigungselement statt als Piston in Form einer Klammer oder stempelförmig zur Auflage auf der Steigbügelfußplatte oder als geschlitzte Glocke zur Befestigung der Gehörknöchelchenprothese auf dem Steigbügel ausgebildet sein.

In weiteren, in der Zeichnung nicht dargestellten Ausführungsformen der Erfindung kann in das Verbindungselement 2c ein Kugelgelenk integriert sein, um eine gewisse postoperative Flexibilität der Gehörknöchelchenprothese 2 zwischen ihren Verbindungsstellen sicherzustellen. Dies wird dann bei der geometrischen Ausgestaltung der Einhängevorrichtungen 6b entsprechend berücksichtigt.

Ebenfalls in der Zeichnung nicht dargestellt sind Ausführungsformen, bei denen die Gehörknöchelchenprothese mit einem aktiven Vibrationsteil eines aktiven, teilweise implantierbaren Hörgeräts verbunden werden kann.

## Patentansprüche

1. Transportvorrichtung (1), die für eine Aufnahme und gegen Herausfallen gesicherte Halterung einer Gehörknöchelchenprothese (2) zur Implantation ins Mittelohr während des Transports zum Operateur eingerichtet ist, wobei die Gehörknöchelchenprothese (2) mindestens Teile eines Gliedes der menschlichen Gehörknöchelchenkette ersetzen oder überbrücken kann, wobei die Gehörknöchelchenprothese (2) an ihrem einen Ende ein erstes Befestigungselement (2a) zur mechanischen Verbindung mit dem Trommelfell oder einem Glied der Gehörknöchetchenkette und an ihrem anderen Ende ein zweites Befestigungselement (2b) zur mechanischen Verbindung mit einem weiteren Glied oder Teilen eines Gliedes der Gehörknöchelchenkette oder direkt mit dem Innenohr sowie ein die beiden Befestigungselemente (2a, 2b) Schall leitend miteinander verbindendes Verbindungselement (2c) aufweist **dadurch gekennzeichnet,**
**dass** die Transportvorrichtung (1) eine flache Grundplatte (5) aus dünnem Metallblech mit einer Dicke von etwa einem halben Millimeter umfasst, in die mindestens eine mit der Grundplatte (5) über feine Stege (6b') verwindbar verbundene Einhängevorrichtung (6b) für die Aufnahme und Halterung der Gehörknöchelchenprothese (2) integriert ist, und dass die Einhängevorrichtung (6b) bei der Fertigung der Transportvorrichtung (1) mit der Oberfläche der Grundplatte (5) fluchtet und zum Einhängen der Gehörknöchelchenprothese (2) gegenüber diesem flach liegenden Fertigungszustand mittels einer plastischen Verformung der Stege (6b') um etwa 90° gegenüber der Grundplatte (5) verdreht werden kann und dann über die Oberfläche der Grundplatte (5) herausragt.

2. Transportvorrichtung nach Anspruch 1, **dadurch gekennzeichnet, dass** die Transportvorrichtung (1) durch Bearbeitung der Grundplatte (5) mittels Laserbehandlung und/oder Anodisierung hergestellt ist.

3. Transportvorrichtung nach einem der vorhergehenden Ansprüche, **dadurch gekennzeichnet, dass** die Grundplatte (5) und die Einhängevorrichtung (6b) gemeinsam aus einem zusammen hängenden Nutzen gefertigt sind.

4. Transportvorrichtung nach einem der vorhergehenden Ansprüche, **dadurch gekennzeichnet, dass** die Transportvorrichtung (1) eine während des Transports die Grundplatte (5) allseitig umhüllende Umverpackung, insbesondere in Form eines Behälters wie einer Schachtel, einer Dose oder eines Kästchens umfasst.

5. Transportvorrichtung nach Anspruch 4, **dadurch gekennzeichnet, dass** die Grundplatte (5) eine Einrichtung zur Transportsicherung aufweist, welche seitlich von der Grundplatte (5) abstehende grätenförmige Stege (7) umfasst, die sich gegen Innenwände der Umverpackung verklemmen oder verspannen lassen.

6. Transportvorrichtung nach einem der vorhergehenden Ansprüche, **dadurch gekennzeichnet, dass** zumindest Teile der Gehörknöchelchenprothese (2) aus einem Material mit Formgedächtnis (= memory effect) hergestellt sind, welche thermisch vorbehandelt und bei der Implantation der Gehörknöchelchenprothese (2) im Mittelohr einer weiteren Form verändernden thermischen Behandlung unterzogen werden, dass eine Vorrichtung zur Ermittlung einer optimierten Leistungseinstellung und/oder Energieapplikations-Stelle für die Bearbeitung der intraoperativ thermisch zu behandelnden Teile der Gehörknöchelchenprothese (2) vorgesehen ist, die eine der Gehörknöchelchenprothese (2) nachgebildete Prothesenattrappe (3) enthält, welche zumindest in denjenigen Bereichen, in denen die zugehörige Gehörknöchelchenprothese (2) intraoperativ thermisch behandelt werden soll, mit der Gehörknöchelchenprothese (2) hinsichtlich Material, geometrischer Gestaltung und Herstellungsverfahren identisch aufgebaut ist, und dass die Transportvorrichtung (1) eine Halterungsvorrichtung aufweist, welche die Prothesenattrappe (3) verschwenkbar aufnehmen kann.

7. Transportvorrichtung nach Anspruch 6, **dadurch gekennzeichnet, dass** die Halterungsvorrichtung für die Prothesenattrappe (3) eine mit der Grundplatte (5) über feine Stege (6a') verwindbar verbundene weitere Einhängevorrichtung (6a) zum Einhängen eines Endes der Prothesenattrappe (3) enthalt.

8. Transportvorrichtung nach Anspruch 6 oder 7, **dadurch gekennzeichnet, dass** die Prothesenattrappe (3) eine Einrastvorrichtung (4) enthält, mit welcher die Prothesenattrappe (3) zumindest in einer Bearbeitungsposition in der Grundplatte (5) fixierbar ist.

9. Transportvorrichtung nach einem der Ansprüche 6 bis 8, **dadurch gekennzeichnet, dass** in die Grundplatte (5) einsteckbare oder in der Grundplatte (5) integrierte Platzhalter-Pins (9a, 9b) vorgesehen sind, die so geformt sind, dass sie die thermisch vorbehandelten, aus Material mit Formgedächtnis bestehenden Teile der Gehörknöchelchenprothese (2) sowie gegebenenfalls die entsprechenden Abschnitte der zugehörigen Prothesenattrappe (3) während des Transports zum Operateur in der gewünschten Geometrie derart fixieren, dass auch bei einer eventuellen Erwärmung während des Transports ungewünschte Formveränderungen verhindert werden.

10. Transportvorrichtung nach einem der vorhergehenden Ansprüche, **dadurch gekennzeichnet, dass** die Grundplatte (5) optisch kodiert und/oder beschriftet ist, und dass die Kodierung oder Beschriftung (8) technische Informationen bezüglich der Gehörknöchelchen-prothese (2) und/oder einer zugehöriger Prothesenattrappe (3) und/oder Herstellerangaben enthält.

11. Transportvorrichtung nach Anspruch 10 und einem der Ansprüche 7 bis 10, **dadurch gekennzeichnet, dass** die technischen Informationen der Kodierung und/oder Beschriftung (8) einen empfohlenen Anfangswert für die elektrische Leistung oder Lichtleistung enthalten, welcher zu Beginn der Ermittlung von optimierten Bearbeitungsparametern der intraoperativ thermisch zu behandelnden Teile der Gehörknöchelchenprothese (2) mit Hilfe einer Testbehandlung der Prothesenattrappe (3) angewendet werden soll.

12. Transportvorrichtung nach einem der vorhergehenden Ansprüche, **dadurch gekennzeichnet, dass** die Grundplatte (5) als Arbeitsplatte zur Bearbeitung der Gehörknöchelchenprothese (2) vor der Implantation ins Mittelohr ausgestaltet ist.

13. Transportvorrichtung nach Anspruch 12, **dadurch gekennzeichnet, dass** die Gehörknöchelchenprothese (2) längenvariabel ist und die Grundplatte (5) als Arbeitsplatte zur Einstellung einer individuell gewünschten Länge der Gehörknöchelchenprothese (2) ausgestaltet ist.

## Claims

1. A transport device (1) equipped to receive and securely hold an ossicle prosthetic (2) in such a way that it cannot fall out, for implantation into the middle ear during transportation to the surgeon, wherein the ossicle prosthetic (2) can replace or bridge at least parts of a link in the human ossicle chain, wherein the ossicle prosthetic (2), at its one end, comprises a first fastening element (2a) for mechanical connection to the eardrum or a link of the ossicle chain and at its other end comprises a second fastening element (2b) for mechanical connection to a further link or parts of a link of the ossicle chain or directly to the inner ear, as well as a connecting element (2c) connecting the two fastening elements (2a, 2b) in a sound-conducting manner, **characterised in that** the transport device (1) comprises a flat base plate (5) made of thin metal sheeting with a thickness of about half a millimetre, which has at least one suspension device (6b) integrated with it for receiving and holding the ossicle prosthetic (2), the suspension device being twistably connected with the base plate (5) via fine stays (6b'), and **in that** the suspension device (6b), during manufacture of the transport device (1), is in alignment with the surface of the base plate (5) and can, in relation to this flatlying manufacturing state, be twisted by about 90° relative to the base plate (5) by means of a plastic deformation of the stays (6b') for suspending the ossicle prosthetic (2) and then protrudes from the surface of the base plate (5).

2. Transport device according to claim 1, **characterised in that** the transport device (1) is produced by working the base plate (5) by means of laser treatment and/or anodisation.

3. Transport device according to one of the preceding claims, **characterised in that** the base plate (5) and the suspension device (6b) are produced together from a continuous blank.

4. Transport device according to one of the preceding claims, **characterised in that** the transport device (1) comprises an overpack, in particular in the form of a container such as a carton, a tin or a small box, which envelops the base plate (5) from all sides during transportation.

5. Transport device according to claim 4, **characterised in that** the base plate (5) comprises a feature for safe transportation comprised of herringbone-shaped stays (7) protruding laterally from the base plate (5), which can be jammed or braced against inner walls of the overpack.

6. Transport device according to one of the preceding claims, **characterised in that** at least parts of the ossicle prosthetic (2) are manufactured from a material with memory effect, which parts are thermally pre-treated and, during implantation of the ossicle prosthetic (2) in the middle ear, are subjected to a further shape-changing thermal treatment, **in that** a device is provided for ascertaining an optimised performance setting and/or energy-application point for working the parts of the ossicle prosthetic (2) which are to be thermally treated intra-operatively, which device includes a prosthetic dummy (3) emulating the ossicle prosthetic (2), which dummy, at least in those areas in which the associated ossicle prosthetic (2) is to be thermally treated intra-operatively, is constructed identically to the ossicle prosthetic (2) as regards material, geometrical design and manufacturing method, and **in that** the transport device (1) comprises a holding device capable of pivotably receiving the prosthetic dummy (3).

7. Transport device according to claim 6, **characterised in that** the holding device for the prosthetic dummy (3) includes a further suspension device (6a) twistably connected with the base plate (5) via fine stays (6a') for suspending one end of the prosthetic dummy (3).

8. Transport device according to claim 6 or 7, **characterised in that** the prosthetic dummy (3) includes a locking device (4) with which the prosthetic dummy (3) is fixable in at least one working position in the base plate (5).

9. Transport device according to one of claims 6 to 8, **characterised in that** placeholder pins (9a, 9b) insertable into the base plate (5) or integrated with the base plate (5) are provided, which are shaped such that they can fix the thermally pre-treated parts of the ossicle prosthetic (2) consisting of a material with memory effect as well as, if required, the corresponding portions of the associated prosthetic dummy (3) during transportation to the surgeon in the desired geometry in such a way as to prevent undesired changes in shape, even if they become hot during transportation.

10. Transport device according to one of the preceding claims, **characterised in that** the base plate (5) is optically coded and/or labelled and **in that** the coding or labelling (8) includes technical information relating to the ossicle prosthetic (2) and/or an associated prosthetic dummy (3) and/or manufacturing data.

11. Transport device according to claim 10 and one of claims 7 to 10, **characterised in that** the technical coding and/or labelling (8) information includes a recommended initial value for the electrical output or light output, which value is to be applied at the start of ascertaining optimised working parameters of the parts of the ossicle prosthetic (2) to be thermally treated intra-operatively with the aid of a test treatment of the prosthetic dummy (3).

12. Transport device according to one of the preceding claims, **characterised in that** the base plate (5) is designed as a work surface for working the ossicle prosthetic (2) prior to implantation into the middle ear.

13. Transport device according to claim 12, **characterised in that** the ossicle prosthetic (2) is variable in length and **in that** the base plate (5) is designed as a work surface for setting an individually desired length of the ossicle prosthetic (2).

## Revendications

1. Dispositif de transport (1) qui est aménagé pour recevoir une prothèse d'osselets d'oreille (2) destinée à une implantation dans l'oreille moyenne et lui assurer un support sécurisé contre tout dégagement au cours du transport à l'opérateur, dans lequel la prothèse d'osselets d'oreille (2) peut remplacer ou ponter au moins des parties d'un segment de la chaîne humaine d'osselets de l'oreille, dans lequel la prothèse d'osselets d'oreille (2) présente, à sa première extrémité, un premier élément de fixation (2a) pour assurer la liaison mécanique avec le tympan ou un segment de la chaîne d'osselets d'oreille et, à son autre extrémité, un deuxième élément de fixation (2b) pour assurer la liaison mécanique avec un autre segment ou des parties d'un segment de la chaîne d'osselets d'oreille ou directement avec l'oreille interne ainsi qu'un élément de fixation (2c) reliant l'un à l'autre de manière conductrice du son les deux éléments de fixation (2a, 2b), **caractérisé en ce que**
le dispositif de transport (1) comprend une plaque de base plate (5) formée d'une tôle métallique mince d'une épaisseur de l'ordre d'un demi-millimètre, à laquelle un dispositif d'accrochage (6b) est intégré pour recevoir et supporter la prothèse d'osselets d'oreille (2), et **en ce que** le dispositif d'accrochage (6b) est aligné, lors de la fabrication du dispositif de transport (1) avec la surface de la plaque de base (5) et peut, pour accrocher la prothèse d'osselets d'oreille (2), être soumis à une déformation plastique des ressauts (6b') d'environ 90 ° par rapport à la plaque de base (5) vis-à-vis de cet état de production à plat, et dépasse ensuite de la surface de la plaque de base (5).

2. Dispositif de transport selon la revendication 1, **caractérisé en ce que** le dispositif de transport (1) est fabriqué par façonnage de la plaque de base (5) par traitement au laser et/ou anodisation.

3. Dispositif de transport selon l'une quelconque des revendications précédentes, **caractérisé en ce que** la plaque de base (5) et le dispositif d'accrochage (6b) sont fabriqués conjointement à partir d'une pièce commune.

4. Dispositif de transport selon l'une quelconque des revendications précédentes, **caractérisé en ce que** le dispositif de transport (1) comprend un conditionnement entourant de toutes parts la plaque de base (5) au cours du transport, en particulier sous la forme d'un réceptacle, tel qu'un carton, une boîte ou une caissette.

5. Dispositif de transport selon la revendication 4, **caractérisé en ce que** la plaque de base (5) présente un dispositif de sécurisation du transport, qui comprend des ressauts (7) en forme d'arêtes saillant latéralement de la plaque de base (5) et qui peuvent se coincer ou s'immobiliser contre les parois internes du conditionnement.

6. Dispositif de transport selon l'une quelconque des revendications précédentes, **caractérisé en ce qu'**au moins des parties de la prothèse d'osselets d'oreille (2) sont fabriquées à partir d'un matériau à effet de mémoire, lesquelles parties sont prétraitées thermiquement et soumises, lors de l'implantation de la prothèse d'osselets d'oreille (2) dans l'oreille moyenne, à une autre forme de traitement thermique modifié, **en ce qu'**il est prévu un dispositif permettant d'obtenir un réglage de capacité optimisé et/ou un point d'application d'énergie pour le façonnage des parties de la prothèse d'osselets d'oreille (2) à traiter thermiquement en cours d'opération, lequel dispositif contient une attrape-prothèse (3) conformée selon la prothèse d'osselets d'oreille (2), laquelle attrape est conçue de manière identique, au moins dans les zones dans lesquelles la prothèse d'osselets d'oreille (2) correspondante doit être traitée par voie thermique en cours d'opération, à la prothèse d'osselets d'oreille (2) en matière de matériau, de conformation géométrique et de procédure de fabrication, et **en ce que** le dispositif de transport (1) présente un dispositif de support qui peut recevoir à pivotement l'attrape-prothèse (3).

7. Dispositif de transport selon la revendication 6, **caractérisé en ce que** le dispositif de support pour l'attrape-prothèse (3) contient un autre dispositif d'accrochage (6a) relié à la plaque de base (5) via de fins ressauts (6a') de manière à pouvoir être déformé pour accrocher une extrémité de l'attrape-prothèse (3).

8. Dispositif de transport selon la revendication 6 ou la revendication 7, **caractérisé en ce que** l'attrape-prothèse (3) contient un dispositif d'encliquetage (4), avec lequel l'attrape-prothèse (3) peut être fixé dans la plaque de base (5) au moins dans une position de façonnage.

9. Dispositif de transport selon l'une quelconque des revendications 6 à 8, **caractérisé en ce qu'**il est prévu des broches de substitution (9a, 9b) qui peuvent être enfichées dans la plaque de base (5) ou intégrées à la plaque de base (5), les broches étant formées de manière qu'elles fixent les parties de la prothèse d'osselets d'oreille (2) prétraitées thermiquement et constituées d'un matériau à effet de mémoire ainsi qu'éventuellement les sections correspondantes de l'attrape-prothèse correspondante (3) au cours du transport à l'opérateur dans la géométrie souhaitée pour que, même au cours d'un échauffement éventuel au cours du transport, des modifications de forme non souhaitées soient empêchées.

10. Dispositif de transport selon l'une quelconque des revendications précédentes, **caractérisé en ce que** la plaque de base (5) est optiquement codée et/ou marquée et **en ce que** le codage ou la marque (8) contient des informations techniques concernant la prothèse d'osselets d'oreille (2) et/ou un attrape-prothèse correspondant (3) et/ou des indications du fabricant.

11. Dispositif de transport selon la revendication 10 et l'une quelconque des revendications 7 à 10, **caractérisé en ce que** les informations techniques du codage et/ou de la marque (8) contiennent une valeur de départ recommandée pour la puissance électrique ou la puissance lumineuse, qui doit être utilisée au début de la détection de paramètres de façonnage optimisés des parties de la prothèse d'osselets d'oreille (2) à traiter thermiquement en cours d'opération à l'aide d'un traitement d'essai de l'attrape-prothèse (3).

12. Dispositif de transport selon l'une quelconque des revendications précédentes, **caractérisé en ce que** la plaque de base (5) se présente sous la forme d'une plaque de travail pour façonner la prothèse d'osselets d'oreille (2) avant l'implantation dans l'oreille moyenne.

13. Dispositif de transport selon la revendication 12, **caractérisé en ce que** la prothèse d'osselets d'oreille (2) est variable en longueur et la plaque de base (5) se présente sous la forme d'une plaque de travail pour régler une longueur individuellement souhaitée de la prothèse d'osselets d'oreille (2).
